# EUROPEAN PATENT APPLICATION

(11) **EP 3 275 994 A1**
(43) Date of publication of application: **31.01.2018**
(21) Application number: 16772300.6
(22) Date of filing: 16.03.2016
(51) Int. Cl.: C12M 3/00, C12Q 1/02, G01N 33/15, G01N 33/50

(54) **HEPATOCYTE CULTURE DEVICE CAPABLE OF ENHANCING ACCUMULATION AND EXCRETION OF HEPATIC METABOLITE IN AND INTO BILE CANALICULUS-LIKE STRUCTURE, AND METHOD FOR EVALUATING CANDIDATE COMPOUND SENSITIVE TO EXCRETION INTO BILE OR BLOOD AND HEPATIC METABOLITE OF SAID CANDIDATE COMPOUND USING SAID HEPATOCYTE CULTURE DEVICE**

(30) Priority: 27.03.2015 JP 2015065826
(71) Applicant: NATIONAL AGRICULTURE AND FOOD RESEARCH ORGANIZATION, Tsukuba-shi, Ibaraki 305-8517 (JP)
(72) Inventor: TAKEZAWA, Toshiaki, Tsukuba-shi, Ibaraki 305-8602 (JP)
(74) Representative: Brann AB
(86) International application number: PCT/JP2016/058320
(87) International publication number: WO 2016/158417

(57) **Abstract**

The present invention is a hepatocyte culture device enhancing accumulation and excretion of a hepatic metabolite in and into a bile canaliculus-like structure, the device including: a first cell culture containing a plurality of hepatocytes; a portable culture vessel in which the bile canaliculus-like structure can be established between the hepatocytes in the first cell culture, and that has, on an outer surface of the culture vessel, a membrane through which a physiologically active substance can pass; and a second cell culture capable of improving excretion activity of a hepatic metabolite in the first cell culture, in which the first cell culture cultured in the portable culture vessel is disposed on the second cell culture to be co-cultured.

## Description

### Technical Field

The present invention relates to a hepatocyte culture device enhancing accumulation and excretion of a hepatic metabolite in and into a bile canaliculus-like structure, and a method for evaluating a candidate compound sensitive to excretion into bile or blood and a hepatic metabolite of the candidate compound using the hepatocyte culture device.

Priority is claimed on Japanese Patent Application No. 2015-065826, filed on March 27, 2015, the content of which is incorporated herein by reference.

### Background Art

If drugs or other compounds are administered orally, these are absorbed in the stomach or small intestine, flow into the blood and enter the liver via blood vessels such as the portal vein. Thereafter, the drugs or other compounds are absorbed and metabolized in the hepatocytes in the liver, but there are two excretion pathways of hepatic metabolites as follows. First is a pathway in which hepatic metabolites are excreted into the bile canaliculus as well as bile, reach the gallbladder, and then excreted out of the body as excrement via the intestine. The other is a pathway in which hepatic metabolites are excreted into sinusoidal blood vessels, reach the kidneys, and then excreted out of the body as urine. It is known transporters that introduce drugs or other compounds into the hepatocytes and transporters for excretion of the hepatic metabolites are different in these two excretion pathways of hepatic metabolites, and the type and amount of hepatic metabolites are also different depending on animals.

In the pharmaceutical industry, drug research and development are in progress while particularly focusing on cytochrome P450 3A4 (CYP3A4). CYP3A4 is a type of cytochrome P450 (CYP) molecular species, is one of the major enzymes that metabolize xenobiotics present in the human body, and accounts for the majority of CYP present in the liver.

When analyzing the drug metabolism by CYP3A4, because frozen human hepatocytes have individual differences in the activity of CYP3A4, cell lines derived from a human liver tumor, so called HypaRG (registered trademark) developed at the French National Institute of Health and Medical Research (INSERM) cell lines are used. It is considered that HepaRG (registered trademark) cells have an average activity in the human liver with respect to CYP3A4.

As an evaluable culture system of a candidate compound sensitive to excretion into bile, a sandwich culture method developed by Dr. Brouwer et al. is the only method at the moment. In this method, the formation of a bile canaliculus-like structure between the hepatocytes is enhanced by sandwich culture of the hepatocytes using an extracellular matrix such as collagen gel. Specifically, a standard medium containing calcium and a medium containing no calcium are prepared, and the hepatocytes are sandwich-cultured in each medium to expose the compound. At this time, in the medium containing no calcium, a bile canaliculus-like structure is not constructed between the hepatocytes, and therefore hepatic metabolites are discharged into the extracellular space. Therefore, by analyzing the difference in hepatic metabolites between the standard medium containing calcium and the medium containing no calcium after compound exposure, it is possible to analyze a candidate compound sensitive to excretion into bile.

PTLs 1 and 2 disclose a method for screening a candidate compound sensitive to excretion into bile, in which a sandwich culture method is employed.

PTL 3 discloses a method for evaluating hepatotoxicity of a test compound, in which a sandwich culture method is employed.

### Citation List

### Patent Literature

[PTL 1] Japanese Patent No. 4451570
[PTL 2] Japanese Patent No. 4951506
[PTL 3] Japanese Unexamined Patent Application, First Publication No. 2013-17411

### Summary of Invention

### Technical Problem

Frozen human hepatocytes have not only individual differences in the activity of CYP3A4 but are also expensive and require costs. It is necessary that the HepaRG (registered trademark) cells be cultured in the presence of dimethyl sulfoxide (DMSO) for about 2 weeks in order to restore the activity of CYP3A4, and culture time is required. In addition, costs are required for purchase.

In the sandwich culture method, a method for culture in a calcium-free environment is employed as a comparison object in order to analyze hepatic metabolites excreted into a bile canaliculus-like structure, and it cannot be said that the results obtained necessarily reflect the environment in vivo. Furthermore, production is performed in a general culture dish in the sandwich culture method, and therefore, it is impossible to perform co-culture on bile duct-derived cells which are cultured as a monolayer in advance.

The present invention has been made in view of the above circumstances and an object thereof is to provide a hepatocyte culture device enhancing accumulation and excretion of a hepatic metabolite in and into a bile canaliculus-like structure, and a method for evaluating a candidate compound sensitive to excretion into bile or blood and a hepatic metabolite of the candidate compound using the hepatocyte culture device.

### Solution to Problem

As a result of intensive research to solve the above problems, the inventors of the present invention have found that a first cell culture containing a plurality of hepatocytes is cultured in a portable culture vessel in which a bile canaliculus-like structure can be established between the hepatocytes in the first cell culture, and that has, on an outer surface of the culture vessel, a membrane through which a physiologically active substance can pass, and then the first cell culture is disposed on a second cell culture capable of improving excretion activity of a hepatic metabolite in the first cell culture so as to be co-cultured therewith, and thereby a hepatocyte culture device enhancing excretion of the hepatic metabolite accumulated in the bile canaliculus-like structure between the hepatocytes and in the hepatocytes in the first cell culture can be obtained in a short period of time at low cost, and therefore have completed the present invention.

That is, the present invention is as follows.
(1) A hepatocyte culture device enhancing accumulation and excretion of a hepatic metabolite in and into a bile canaliculus-like structure, the device including: a first cell culture containing a plurality of hepatocytes; a portable culture vessel in which the bile canaliculus-like structure can be established between the hepatocytes in the first cell culture, and that has, on an outer surface of the culture vessel, a membrane through which a physiologically active substance can pass; and a second cell culture capable of improving excretion activity of a hepatic metabolite in the first cell culture, in which the first cell culture cultured in the portable culture vessel is disposed on the second cell culture to be co-cultured.
(2) The hepatocyte culture device according to (1), in which the portable culture vessel improves the metabolic activity of the first cell culture.
(3) The hepatocyte culture device according to (1) or (2), in which the excretion activity of the hepatic metabolite is the activity of excreting the hepatic metabolite accumulated in the bile canaliculus-like structure between the hepatocytes and in the hepatocytes in the first cell culture.
(4) The hepatocyte culture device according to any one of (1) to (3), in which the hepatocytes are cultured hepatocytes derived from normal liver tissues, liver cancer tissues, or stem cells which are selected from the group consisting of humans, rats, monkeys, apes, cats, dogs, pigs, cows, sheep, horses, chickens, and ducks.
(5) The hepatocyte culture device according to any one of (1) to (4), in which the second cell culture is a cultured cell derived from an epithelium, mesenchyme, or an endothelium which are selected from the group consisting of humans, rats, monkeys, apes, cats, dogs, pigs, cows, sheep, horses, chickens, and ducks, and is a cell selected from the group consisting of intrahepatic bile duct epithelial cells, extrahepatic bile duct epithelial cells, vascular endothelial cells, fibroblasts, and culture supernatant fluids of these cells.
(6) The hepatocyte culture device according to any one of (1) to (5), in which the membrane through which a physiologically active substance can pass contains an extracellular matrix component to induce a gelation.
(7) The hepatocyte culture device according to (6), in which the extracellular matrix component to induce a gelation is collagen.
(8) A method for evaluating a candidate compound sensitive to excretion into bile or blood, and a hepatic metabolite of the candidate compound using hepatocytes, in which a first cell culture containing a plurality of hepatocytes, a portable culture vessel in which a bile canaliculus-like structure can be established between the hepatocytes in the first cell culture, and that has, on an outer surface of the culture vessel, a membrane through which a physiologically active substance can pass, and a second cell culture capable of improving excretion activity of a hepatic metabolite in the first cell culture, are included, the method including: (a) a step of exposing the candidate compound to the first cell culture cultured in the portable culture vessel; (b) a step of culturing the first cell culture to which the candidate compound is exposed by disposing the culture on each of a vessel containing only a culture medium and a vessel containing the second cell culture; (c) a step of checking a change in the bile canaliculus-like structure of the first cell culture, and recovering a substance excreted into the portable culture vessel and a substance excreted into each of the vessel containing only the culture medium and the vessel containing the second cell culture; and (d) a step of analyzing the recovered substances for an excreted hepatic metabolite and an amount thereof.
(9) The evaluation method according to (8), in which the portable culture vessel improves metabolic activity of the first cell culture.
(10) The evaluation method according to (8) or (9), in which the excretion activity of the hepatic metabolite is activity of excreting the hepatic metabolite accumulated in the bile canaliculus-like structure between the hepatocytes and in the hepatocytes in the first cell culture.

### Advantageous Effects of Invention

According to a hepatocyte culture device of the present invention, it is possible to obtain a substance metabolized by hepatocytes in the environment reflecting in vivo in a short period of time at low cost. It is possible to collectively recover hepatic metabolites excreted into a bile canaliculus-like structure established between the hepatocytes without destroying tight junctions between the hepatocytes.

According to a method for evaluating a candidate compound sensitive to excretion into bile or blood, and a hepatic metabolite of the candidate compound using the hepatocyte culture device of the present invention, it is possible to evaluate types and amount of hepatic metabolite metabolized and excreted into bile or blood by activated drug-metabolizing enzymes, and to further evaluate hepatotoxicity of the candidate compound and a hepatic metabolite of the candidate compound in a short period of time at low cost.

### Brief Description of Drawings

FIG. 1 is a perspective view illustrating one embodiment of a portable culture vessel in the present invention.
FIG. 2 is a view illustrating one embodiment of a rapid activation process of a liver function by a first cell culture in the present invention. In FIG. 2, P is an enlarged schematic view of HepG2 cells.
FIG. 3 is a graph illustrating albumin production level, urea synthesis level, and CYP3A4 activity level of HepG2 cells after rapid activation of a liver function in Example 1, and those of HepG2 cells cultured in a solid phase and a liquid phase as a comparative example.
FIG. 4 is a schematic view illustrating an operation procedure in Example 1. In FIG. 4, Q is an enlarged schematic view of HepG2 cells and TFK-1 cells.
FIG. 5 is a picture taken with a digital microscope camera by observing HepG2 cells, and Fluorescein (excitation wavelength: 490 nm, emission wavelength: 514 nm) metabolized by the cells with a phase contrast microscope (upper row) and a fluorescence microscope (lower row) in Observations A to C of Example 1.
FIG. 6 is a schematic view illustrating experimental conditions i) to iv) in Example 2.
FIG. 7 is a graph illustrating fluorescence intensities of Fluorescein excreted into a chamber (upper side) and a dish (lower side) in homo-culture of HepG2 cells alone, and fluorescence intensities of Fluorescein excreted into the chamber (upper side) and the dish (lower side) in co-culture of HepG2 cells and TFK-1 cells, at culture time of 3 minutes, 30 minutes, and 60 minutes, in Example 3.
FIG. 8 is a graph illustrating fluorescence intensities of Fluorescein excreted into the chamber (upper side) and the dish (lower side) in the homo-culture of HepG2 cells alone, and fluorescence intensities of Fluorescein excreted into the chamber (upper side) and the dish (lower side) in the co-culture of HepG2 cells and TFK-1 cells, at culture time of 3 minutes, 30 minutes, 60 minutes, and 180 minutes, in Example 4.
FIG. 9 is a picture taken with a digital microscope camera by observing changes of Fluorescein remaining in the cytoplasm of HepG2 cells and Fluorescein accumulated in a bile canaliculus-like structure established between the cells of HepG2 cells over time with a fluorescence microscope with respect to a set in which only HepG2 cells are homo-cultured in a dish containing a culture medium and a set in which HepG2 cells are co-cultured with TFK-1 cells, at culture time of 0 hours, 1 hour, 3 hours, and 23 hours, in Example 5.
FIG. 10 is a graph illustrating fluorescence intensities of Fluorescein excreted into the chamber (upper side) and the dish (lower side) in the homo-culture of HepG2 cells alone, fluorescence intensities of Fluorescein excreted into the chamber (upper side) and the dish (lower side) in the co-culture of HepG2 cells and TFK-1 cells, and fluorescence intensities of Fluorescein excreted into the chamber (upper side) and the dish (lower side) in co-culture of HepG2 cells and NHDFs in Example 6.
FIG. 11A is a schematic view illustrating membrane proteins present in a hepatocyte and a bile canaliculus.
FIG. 11B is an image illustrating results of subjecting MRP2 and BSEP to immunostaining with respect to a set in which HepG2 cells are seeded directly in the dish and a set in which HepG2 cells are seeded in a hydrogel membrane chamber to be activated in Example 7.

### Description of Embodiments

Hereinafter, the present invention will be described in more detail while showing an embodiment, but the present invention is not limited to the following embodiments at all.

### [First Cell Culture]

In a hepatocyte culture device of the present invention, a first cell culture contains a plurality of hepatocytes. The hepatocytes are preferably cultured hepatocytes derived from normal liver tissues, liver cancer tissues, or stem cells (ES cells, iPS cells, mesenchymal stem cells, and the like) which are selected from the group consisting of humans, rats, monkeys, apes, cats, dogs, pigs, cows, sheep, horses, chickens, and ducks, more preferably frozen human hepatocytes, furthermore preferably cell lines derived from human liver tumors, and most preferably HepG2 cells which are one of cell lines derived from human liver cancer. HepG2 cells are inexpensive compared to frozen human hepatocytes and HepaRG (registered trademark) cells, which are cell lines derived from human liver tumors. Furthermore, it is possible to raise the activity of CYP3A4 up to about 1/2 of that of HepaRG cells expressing the average activity in human liver in a short period of 3 days by the rapid activation of a liver function to be described later.

### [Portable Culture Vessel Having Membrane through Which Physiologically Active Substance Can pass]

In the hepatocyte culture device of the present invention, a portable culture vessel having a membrane on an outer surface thereof through which a physiologically active substance can pass is used in order to establish a bile canaliculus-like structure between hepatocytes in the first cell culture.

In the present specification, the "outer surface" means every surface in contact with the outside of the culture vessel and includes a bottom surface, a side surface, and a ceiling surface. Among these, in the hepatocyte culture device of the present invention, a membrane through which a physiologically active substance can pass is preferably provided on the bottom surface.

A specific embodiment of the portable culture vessel having a membrane through which a physiologically active substance can pass is described below.

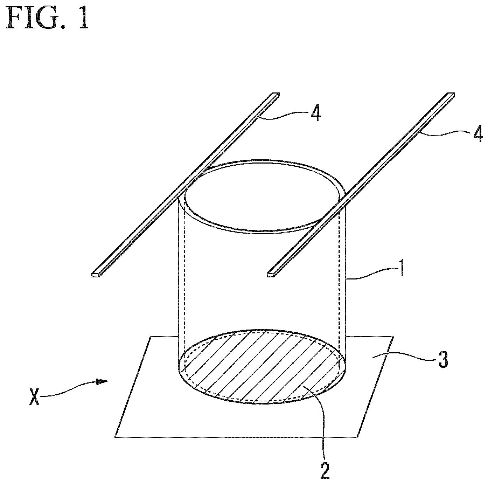

FIG. 1 is a perspective view illustrating one embodiment of the portable culture vessel having a membrane through which a physiologically active substance can pass in the present invention. A specific configuration of a portable culture vessel X and a manufacturing method thereof are disclosed in Japanese Unexamined Patent Application, First Publication No. 2012-115262. A tubular frame body 1 has a space for holding cells therein. As a material of the frame body 1, a material suitable for cell culture can be appropriately selected. The shape of the frame body 1 is not limited, and examples thereof include a cylindrical shape or a rectangular tube shape. Specifically, as the frame body 1, a cylindrical tube made of acrylic or polystyrene can be preferably exemplified.

A membrane 2 allowing a physiologically active substance to pass through is adhered to be fixed to one open end surface of the frame body 1 with an adhesive. A shape of the open-end surface is not particularly limited, and examples thereof include a planar shape or a stepped shape, a tapered shape, a groove shape, and the like, but the planar shape is preferable. Furthermore, in a case of using the membrane 2 overlapped with a film 3, the membrane 2 adheres to the frame body 1, but the film 3 can be peeled off to be removed. Furthermore, a locking portion 4 protruding to the outside of a carrier 1 is disposed on the outer peripheral edge portion of the open-end surface facing the end surface of the frame body 1 to which the membrane 2 is coated and fixed. For example, by putting the culture vessel X on another vessel larger than the portable culture vessel X, it is possible to hold the culture vessel X in the vessel. The locking portion 4 is not limited to the form illustrated in FIG. 1, but can be in the form of a rod shape, a flange shape, and the like for example with a plastic material or the like.

The adhesive for adhering and fixing, to the carrier 1, the membrane 2 through which a physiologically active substance can pass, can be appropriately selected in consideration of adhesiveness and cytotoxicity, and specific examples preferably include a urethane adhesive. For example, a rubber type, a cyan acrylate type, and an acrylic type may exhibit cytotoxicity in some cases, and a hot melt type may heat denature the membrane 2 through which a physiologically active substance can pass, which is not preferable. In addition, examples of a method for adhering and fixing the membrane 2 through which a physiologically active substance can pass to the frame body 1 include a method for adhering and fixing the membrane 2 through which a physiologically active substance can pass to the frame body 1 by interposing a double-faced tape therebetween, a method for heat welding the membrane 2 through which a physiologically active substance can pass to the frame body 1 using a heat sealer or a hot plate, an ultrasonic wave, a laser, or the like.

Examples of the film 3 from which the membrane 2 through which a physiologically active substance can pass can be peeled include a non-absorbable plastic thin film such as polyethylene, polyethylene terephthalate (PET), polypropylene, Teflon (registered trademark), silicone, Saran wrap (registered trademark), vinyl, and parafilm, and a silicone-coated PET thin film is particularly preferable. The silicone-coated PET thin film is excellent in water repellency and realizes appropriate adhesiveness and peelability with respect to the membrane through which a physiologically active substance can pass (refer to Japanese Patent Application No. 2014-109499).

In the present invention, the "physiologically active substance" means a component of a culture medium and a product substance of cultured cells, and includes from a substance with a small molecular weight to a substance with a large molecular weight of 20,000 or more. Example thereof include various medicines including antibiotics, cell growth factors, differentiation-inducing factors, cell adhesion factors, antibodies, enzymes, cytokines, hormones, lectins, or fibronectin, entactin, osteopoietin, and the like, as a non-gelatinized extracellular matrix component.

As a component constituting the membrane through which the physiologically active substance can pass, a hydrogel is preferable. The "hydrogel" refers to a substance in which a polymer has a network structure by chemical bonding and contains a large amount of water in the network, and more specifically refers to a gelation substance obtained by introducing cross-linkages to an artificial material of a synthetic polymer or a polymer derived from a natural product.

As the polymer derived from a natural product used for production of the hydrogel, it is preferable to contain an extracellular matrix component to induce gelation. Examples of the extracellular matrix component to induce gelation include collagen (type I, type II, type III, type V, type XI, and the like) and basement membrane components (product name: Matrigel) reconstituted from mouse EHS tumor extract (including type IV collagen, laminin, heparan sulfate proteoglycan, and the like), fibrin, glycosaminoglycan, hyaluronic acid, proteoglycan, and the like. As other polymers derived from a natural product, it is also possible to use gelatin, agar, agarose, and the like. It is possible to produce the hydrogel by selecting a component such as salt optimum for each gelation, concentration, pH, and the like thereof. By combining with a raw material, it is possible to obtain the membrane through which the physiologically active substance can pass, which mimics various in vivo tissues.

As the synthetic polymer used for production of the hydrogel, there are polyacrylamide, polyvinyl alcohol, methyl cellulose, polyethylene oxide, poly(II-hydroxyethyl methacrylate)/polycaprolactone, and the like. It is also possible to produce the hydrogel by using two or more of these polymers. The amount of hydrogel can be adjusted in consideration of the thickness of the membrane through which the physiologically active substance can pass to be produced.

Among these, collagens are preferable as a raw material for the hydrogel, and furthermore, native collagen is a more preferable raw material among collagens. Because atelocollagen gel is generally inferior in strength than the native collagen gel of the same concentration, by using the native collagen, it is possible to manufacture the portable culture vessel having the membrane through which the physiologically active substance can pass, which is excellent in handling.

The membrane through which the physiologically active substance can pass can be inserted into a culture vessel to be used as a cell culture carrier for culturing animal cells. Examples of the animal cells to be cultured include primary cultured cells, cell lines, fertilized eggs, and cells into which foreign genes have been introduced into these cells. Furthermore, these cells may be undifferentiated stem cells, cells in differentiation, terminally differentiated cells, and dedifferentiated cells. Examples of means for starting culture of these cells include cell suspension, chopped pieces of tissue, fertilized eggs, or seeding of multicellular aggregates reconstructed three-dimensionally. In other words, adherent cells that can be cultured by an existing method can be cultured on a membrane through which the physiologically active substance can pass.

In addition, one or more kinds of the animal cells described above can be cultured on both sides of the membrane through which the physiologically active substance can pass as well as one side thereof. Furthermore, in the both-side culture, it is possible to culture heterologous cells on each side. Particularly, by culturing epithelial cells on one side of the membrane through which the physiologically active substance can pass, and mesenchymal cells on the other side, a transdermal absorption model an intestinal tract absorption model, and the like having epithelial mesenchymal interaction becomes possible, or, by culturing vascular endothelial cells on one side and cancer cells on the other side, cell function assays such as an angiogenesis model and a cancer infiltration model become possible.

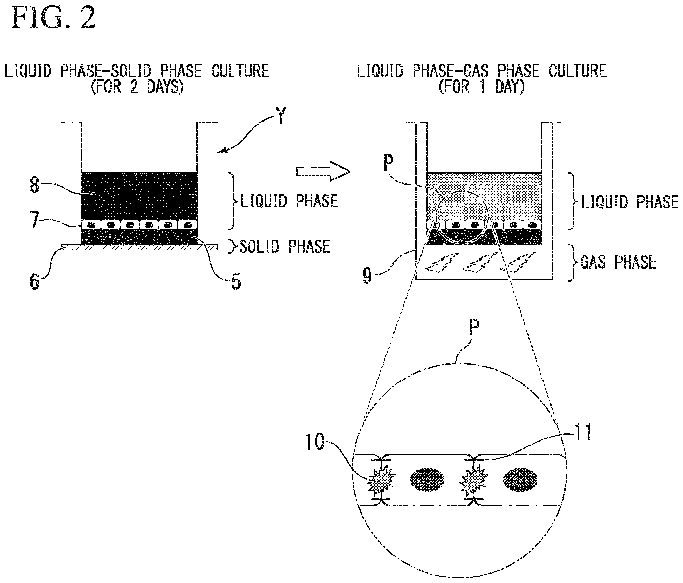

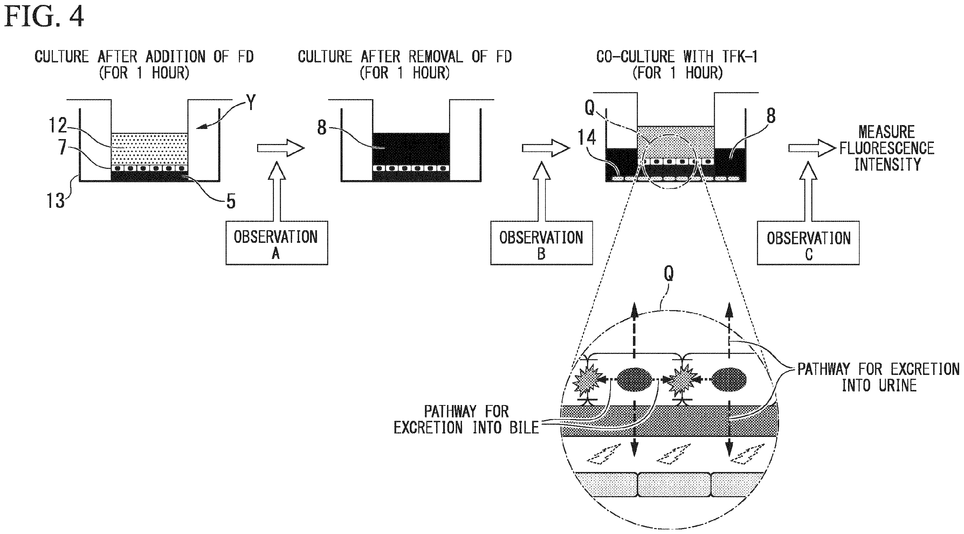

As illustrated in FIG. 2 and FIG. 4, the portable culture vessel having the membrane through which the physiologically active substance can pass according to the present invention can be moved in a state where a culture such as a cell is retained therein so as to carry out culture in different environments such as "liquid phase-membrane-solid phase", "liquid phase-membrane-gas phase", "liquid phase-membrane-liquid phase", and the like.

With such portability, it is easy to construct a hepatocyte culture device enhancing accumulation and excretion of a hepatic metabolite in and into a bile canaliculus-like structure which will be described later.

### [Rapid Activation of Liver Function in First Cell Culture]

In the present invention, it is necessary that the first cell culture be cultured by using the portable culture vessel having the membrane through which the physiologically active substance can pass, and thereby a liver function is activated in order to establish a bile canaliculus-like structure between the hepatocytes in the first cell culture. The activation of a liver function means enhancement in the metabolic activity of the hepatocytes, and it is possible to enhance the metabolic activity of the hepatocytes by culturing the first cell culture in the portable culture vessel.

The rapid activation of a liver function in first cell culture is shown in the specific embodiment as below.

FIG. 2 is a view illustrating one embodiment of a rapid activation process of a liver function by the first cell culture in the present invention. The first cell culture to be used may be the hepatocytes described above, but a case where HepG2 cells are used will be described.

First, the first cell culture is suspended in a culture medium, and seeded in the portable culture vessel having the membrane through which the physiologically active substance can pass.

In the present invention, as the "culture medium", there is no limitation as long as it is a medium generally used for culturing cells, and examples thereof include Dulbecco's Modified Eagle's Medium (DMEM), Minimum Essential Media (MEM), Iscove's Modified Dulbecco's Medium (IMDM), Glasgow's Minimum Essential Medium (GMEM), and the like, but the medium can be appropriately changed in accordance with cells to be used. At this time, from the fact that it takes time to adhere the cells to the membrane on the inner bottom surface of the portable culture vessel and there is a case where the cells tend to adhere and aggregate, a state where the bottom surface is flat and not bent is preferable.

Next, in a state where the film adheres to the lower surface, that is, in a state of "liquid phase-membrane-solid phase", the cells are cultured until the cells become confluent. Culture time varies depending on the cells to be used, but for example, it is preferable that HepG2 cells be cultured for about 2 days.

After the cells became confluent, the film on the lower surface is peeled off, and by putting the portable culture vessel on a vessel with a capacity larger than that of the portable culture vessel, a lower portion of the membrane becomes a gas phase, and the culture is further carried out in a state of "liquid phase-membrane-gas phase". Culture time is preferably about 20 hours to 24 hours.

In order to activate a liver function, it usually takes 10 days to 14 days for frozen human hepatocytes, or HepaRG (registered trademark) cell lines or hepatocytes derived from human iPS cells, whereas for HepG2 cells, a liver function is activated in a short period of 3 days by making the cells be in a confluent state and culturing the cells in a state of "liquid phase-membrane-gas phase".

In the present invention, the "liver function" indicates albumin and urea synthesis level, and pharmacokinetic-related functions, for example, the activity of CYP3A4, and regarding the albumin and urea synthesis level, the liver function is activated up to the level same as that of general hepatocytes. Furthermore, the activity of CYP3A4 can be increased to about 1/2 of that of HepaRG cells expressing the average activity in human liver.

In FIG. 2, P is an enlarged schematic view of HepG2 cells, and a bile canaliculus-like structure and a tight junction are established between the cells. Regarding the hepatocytes, the cells are generally bound to each other by tight junctions, gaps formed between the cells are connected in a tubular shape to become a bile canaliculus, which leads to a bile duct.

Although details of a process by which the hepatocytes are activated are not known, oxygen supplied through the membrane from the gas phase makes it possible for the cells to grow well under aerobic culture conditions despite the high cell density. As a result, it is presumed that the reason is that sufficient exhibition of the self-assembly ability of hepatocytes became possible under the culture conditions.

### [Second Cell Culture]

In the hepatocyte culture device of the present invention, the second cell culture may be a normal cultured cell group, a feeder cell group treated with mitomycin C, a dead cell group fixed with methanol or the like, or a conditioned culture medium of a normal cultured cell group as long as the second cell culture can increase the excretion activity of the hepatic metabolite in the first cell culture. For example, the second cell culture is a cultured cell derived from an epithelium, mesenchyme, or an endothelium which is selected from the group consisting of humans, rats, monkeys, apes, cats, dogs, pigs, cows, sheep, horses, chickens, and ducks, and examples thereof include intrahepatic bile duct epithelial cells, extrahepatic bile duct epithelial cells, vascular endothelial cells, fibroblasts, culture supernatant fluids of these cells, and the like.

The excretion activity of the hepatic metabolite means the activity of excreting the hepatic metabolite accumulated in the bile canaliculus-like structure between the hepatocytes and in the hepatocytes in the first cell culture. An increase or a decrease in the excretion activity of the hepatic metabolite in the first cell culture can be determined by analyzing changes in the amount and the type of metabolized and excreted hepatic metabolites using the hepatocyte culture device described later.

### [Hepatocyte Culture Device]

The hepatocyte culture device of the present invention includes a first cell culture containing a plurality of hepatocytes; a portable culture vessel in which the bile canaliculus-like structure can be established between the hepatocytes in the first cell culture, and that has, on an outer surface of the culture vessel, a membrane through which a physiologically active substance can pass; and a second cell culture capable of improving excretion activity of a hepatic metabolite in the first cell culture, in which the first cell culture cultured in the portable culture vessel is disposed on the second cell culture to be co-cultured.

FIG. 4 is a schematic view illustrating an operation procedure in Example 1 described later. It is preferable that a vessel such as a petri dish containing the second cell culture be bigger than the portable culture vessel and be large enough to be placed on the portable culture vessel. As a culture medium to be used at this time, there is no limitation as long as it is a medium generally used for culturing cells, similarly to the first cell culture.

In FIG. 4, Q is an enlarged schematic view of HepG2 cells, which is one of the cell lines derived from human liver cancer, and TFK-1 cells, which is one of the cell lines derived from human bile duct cancer. At this time, a total amount of the excreted hepatic metabolite (excretion amount in the upper side + excretion amount in the lower side) and a rate of the excretion amount in the lower side are increased compared to a case of non-co-culture with TFK-1 cells, which is the second cell culture. This is attributable to the fact that excretion of the hepatic metabolite accumulated in the bile canaliculus-like structure between the hepatocytes and in the hepatocytes in the first cell culture is enhanced by co-culture with the second cell culture. In a case where the hepatic metabolite is excreted not only into bile but also into the urine, the hepatic metabolite is also excreted from the cytoplasm of the hepatocytes as well as from the bile canaliculus-like structure.

Therefore, by co-culturing the first cell culture with the second cell culture, excretion amount of hepatocyte metabolite increases.

In the hepatocyte culture device of the present invention, as a time for co-culturing the first cell culture with the second cell culture,3 minutes or more is preferable, 30 minutes or more is more preferable. From the viewpoint of growth state of the culture, co-culture time is preferably within 9 days, and co-culture time is more preferably within 2 days for rapidly evaluating excretion of the metabolite in the hepatocytes, and toxicity of a candidate compound sensitive to excretion into bile or blood, and a hepatic metabolite of the candidate compound by using the hepatocyte culture device of the present invention.

As temperature conditions for co-culturing, there is no limitation as long as it is a temperature generally recommended for cell culture, and as a lower limit value, 35°C or more is preferable, and 37°C or more is more preferable. As an upper limit value, 40°C or less is preferable, and 37°C or less is more preferable.

### [Method for Evaluating Candidate Compound Sensitive to Excretion into Bile or Blood and Hepatic Metabolite of Candidate Compound]

In a method for evaluating a candidate compound sensitive to excretion into bile or blood, and a hepatic metabolite of the candidate compound of the present invention, a first cell culture containing a plurality of hepatocytes, a portable culture vessel in which a bile canaliculus-like structure can be established between the hepatocytes in the first cell culture, and that has, on an outer surface of the culture vessel, a membrane through which a physiologically active substance can pass, and a second cell culture capable of improving excretion activity of a hepatic metabolite in the first cell culture, are included, and the method includes steps (a) to (d).
(a) A step of exposing the candidate compound to the first cell culture cultured in the portable culture vessel.
(b) A step of culturing the first cell culture to which the candidate compound is exposed by disposing the culture on each of a vessel containing only a culture medium and a vessel containing the second cell culture.
(c) A step of checking a change in the bile canaliculus-like structure of the first cell culture, and recovering a substance excreted into the portable culture vessel and a substance excreted into each of the vessel containing only the culture medium and the vessel containing the second cell culture.
(d) A step of analyzing the recovered substances for an excreted hepatic metabolite and an amount thereof.

In the present invention, the "candidate compound" indicates that every compound has a possibility of being sensitive to excretion into bile or blood, and examples thereof include xenobiotics such as drugs and other therapeutic agents, carcinogenic substances, and environmental pollutants, and substances in vivo such as steroids, fatty acids, and prostaglandins.

In step (a), the candidate compound can be exposed to the first cell culture by dispersing the compound in the culture medium, or the like. Exposure time depends on the candidate compound, and about 1 hour to 24 hours is preferable, but it is more preferable to determine the time with consideration of the rate of absorption, metabolism, and excretion of the candidate compound. After the exposure, washing is performed with a balanced salt solution or the like, and the candidate compound not introduced into the first cell culture is removed. At this time, the introduced candidate compound and the hepatic metabolite thereof are present in the hepatocytes, but excretion of the hepatic metabolite is already started by the candidate compound. Therefore, if the first cell culture is subsequently cultured using a fresh culture medium after removing the culture medium containing the candidate compound and performing washing with the balanced salt solution or the like, the introduced candidate compound is gradually metabolized. In a case where the hepatic metabolite is excreted not only into bile but also into the urine, the excretion of the hepatic metabolite is accumulated in the bile canaliculus-like structure, and the hepatic metabolite is also excreted into the culture medium from the cytoplasm of the hepatocytes. The hepatic metabolite accumulated in the bile canaliculus-like structure is not excreted into the culture medium if the bile canaliculus-like structure has a closed structure.

In step (b), the first cell culture to which the candidate compound is exposed is co-culture with the second cell culture, and therefore a total amount of the excreted hepatic metabolite (excretion amount in the upper side + excretion amount in the lower side) and a rate of the lower side excretion thereof are increased. The hepatic metabolite was also excreted into the first cell culture cultured in a vessel containing only a culture medium, but a total excretion amount (excretion amount in the upper side + excretion amount in the lower side) and a rate of the lower side excretion are small compared to the case of co-culture with the second cell culture. The excretion amount in the upper side indicates the amount of hepatic metabolite excreted into, for example, the upper side of the first cell culture cultured in the portable culture vessel in FIG. 4, that is, into the portable culture vessel. The excretion amount in the lower side indicates the amount of hepatic metabolite excreted into, for example, the lower side of the first cell culture cultured in the portable culture vessel, that is, into the vessel of the outside of the portable culture vessel by permeating the membrane of the portable culture vessel through which a physiologically active substance can pass.

The culture time and the temperature condition at this time are as described above.

In step (c), in a case of the first cell culture alone, 1 day or more are required for the reduction in the hepatic metabolite accumulated in the bile canaliculus-like structure, and because it is considered that the bile canaliculus-like structure retains almost a closed structure, most of the substances excreted into the portable culture vessel and the substances excreted into the vessel containing only the culture medium are generally excreted in the urine in vivo (refer to Q of FIG. 4). In a case of co-culture of the first cell culture with the second cell culture, the reduction in the hepatic metabolite accumulated in the bile canaliculus-like structure becomes noticeable within 1 hour, and it is considered that the bile canaliculus-like structure is open. If the liver structure of the living body is reflected, an opening of the bile canaliculus-like structure is only the lower side where a bile duct epithelium model of the second cell culture exists.

Therefore, most of the substances excreted into the portable culture vessel include substances generally excreted in the urine in vivo, or the substances excreted into the vessel containing the second cell culture include substances excreted from the lower side of the cytoplasm of the first cell culture and substances excreted from the bile canaliculus-like structure of the first cell culture, collectively include substances excreted in the urine in vivo and substances excreted into the bile in general. If the liver structure of the living body is not reflected, the opening of the bile canaliculus-like structure is only the upper side, or both sides of the upper side and the lower side. Therefore, the substances excreted into the portable culture vessel include substances excreted from the upper side of the cytoplasm of the first cell culture and substances excreted from the bile canaliculus-like structure of the first cell culture, and collectively include substances excreted in the urine in vivo and substances excreted into the bile in general. If the opening of the bile canaliculus-like structure is only the upper side, the substances excreted into the vessel containing the second cell culture only include substances excreted from the lower side of the cytoplasm of the first cell culture, which means the substances include substances excreted in the urine in vivo. If the opening of the bile canaliculus-like structure is both sides of the upper side and the lower side, the substances include substances excreted from the lower side of the cytoplasm of the first cell culture and substances excreted from the bile canaliculus-like structure of the first cell culture, which means the substances collectively include substances excreted in the urine in vivo and substances excreted into the bile in general.

In a case where the candidate compound sensitive to excretion into bile and a hepatic metabolite of the candidate compound have hepatotoxicity specific to the bile canaliculus-like structure, reduction and destruction in the bile canaliculus-like structure of the first cell culture occur, and therefore, the excretion pathway cannot be evaluated. However, hepatotoxicity specific to the bile canaliculus-like structure can be evaluated by quantifying the changes such as reduction or destruction of the bile canaliculus-like structure.

The excreted substances are obtained by recovering a culture medium in the portable culture vessel, and a culture medium in a vessel containing only the culture medium and in a vessel containing the second cell culture. At this time, by adjusting the amount of culture medium to be added to each culture vessel in advance, a total amount of excreted substance obtained in step (d) below can be compared.

In step (d), the excreted hepatic metabolite and the amount thereof can be analyzed by labeling the candidate compound in advance. It is preferable to perform labeling by using compounds that can be easily detected using standard detection techniques such as fluorescence or chemiluminescence spectroscopy, scintillation spectroscopy, chromatography, liquid chromatography/mass spectrometry (LC/MS), and colorimetric analysis. Therefore, labeled compounds include fluorogenic or fluorescent compounds, chemiluminescent compounds, colorimetric compounds, UV/VIS absorbing compounds, radioactive substances, and combinations thereof, but are not limited thereto.

The hepatotoxicity of the candidate compound sensitive to excretion into bile or blood and a hepatic metabolite of the candidate compound can be evaluated by quantifying an indicator substance of liver injury such as deviation enzymes including LDH, GOT, and GPT contained in the culture medium in the portable culture vessel, and the culture medium in a vessel containing only the culture medium and in a vessel containing the second cell culture.

Hereinafter, the present invention will be described according to examples, but the present invention is not limited to the following examples.

### Examples

### [Rapid Activation of Liver Function by HepG2 Cell]

HepG2 cells (purchased from RIKEN BioResource Center, RCB1648, Lot.004) suspended in 0.5 mL DMEM culture medium containing 10% FBS, 20 mM HEPES, 100 units/mL penicillin, and 100 µg/mL streptomycin were seeded at 5 × 10⁴ cells/cm² and cultured for 2 days in a hydrogel membrane chamber (ad-MED Vitrigel (registered trademark) manufactured by KANTO KAGAKU) to which a detachable silicone-coated PET film is attached. Thereafter, the film on a bottom surface of the hydrogel membrane chamber was detached, the chamber was mounted in a well of a 12-well plate, and culture was carried out for 1 day in a state where the bottom surface is in contact with air (refer to FIG. 2). The 0.5 mL culture medium was exchanged daily after seeding.

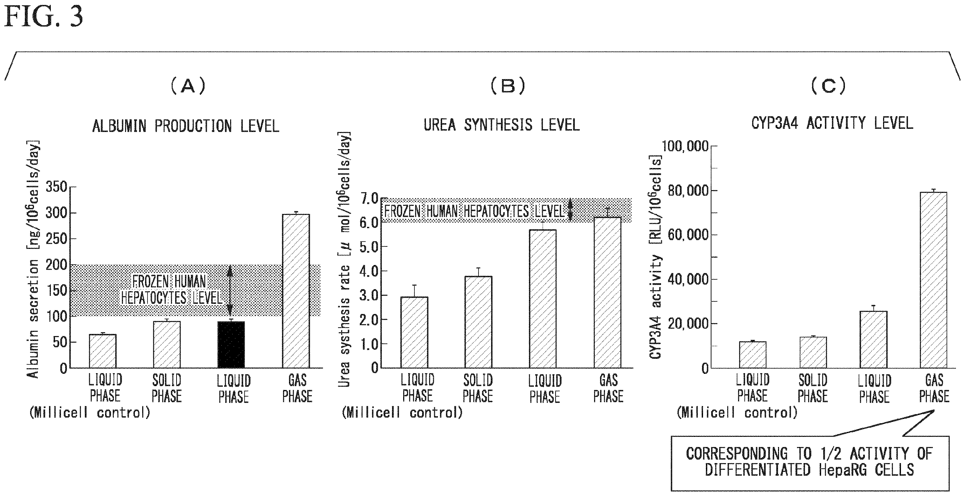

As a result of investigating the liver function of HepG2 cells, the function had the ability to synthesize albumin and urea, and the activity of CYP3A4 was about 1/2 of that of HepaRG cells expressing the average activity in human liver (refer to FIG. 3). In FIG. 3, HepG2 cells cultured in the culture medium were used using Millicell (registered trademark) cell culture inserts as a control. As a comparative example, shown are a solid phase in which the cells were cultured without detaching the film of the hydrogel membrane chamber, and a liquid phase in which the chamber was mounted in a well of the 12-well plate containing the culture medium so as to culture the cells. After culture for 3 days respectively, albumin production level was measured using Human Albumin ELISA Quantitation Set as a measurement kit, urea synthesis level was measured using urea nitrogen test Wako as a measurement kit, and CYP3A4 activity level was measured using P450-Glo CYP3A4 assay with Luciferin-IPA as a measurement kit. It was checked that the liver function of HepG2 cells was activated quickly (3 days) by using the hydrogel membrane chamber.

### [Example 1]

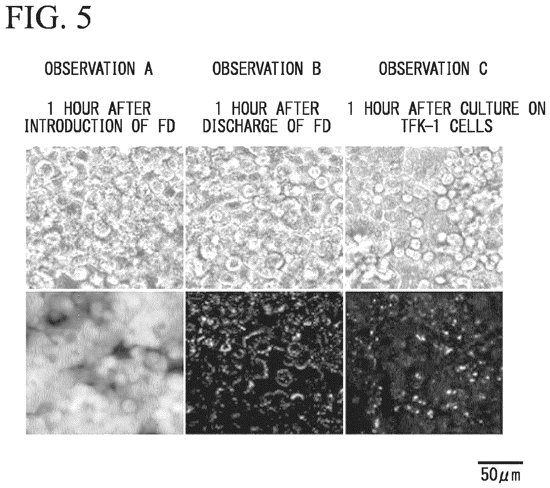

[1] After removing the culture medium of the hydrogel membrane chamber containing the activated HepG2 cells, 0.5 mL of the culture medium containing 250 µg/mL Fluorescein diacetate (FD) was added, and then culture was carried out at 37°C for 1 hour in a CO₂ incubator (refer to FIG. 4 and Observation A of FIG. 5).
   Although FD does not fluoresce as it is, green fluorescence (excitation wavelength: 490 nm, emission wavelength: 514 nm) of Fluorescein was observed by ester bond breakage due to esterase activity in hepatocytes.
[2] After removing the culture medium containing the FD remaining in the chamber, washing was performed twice with 0.5 mL of Hank's Balanced Salt Solution (Hank's balanced salt solution: HBSS), 0.5 mL of the culture medium was added to the chamber, and then culture was carried out at 37°C for 1 hour in a CO₂ incubator (refer to Observation B of FIG. 5).
[3] Subsequently, after removing the culture medium remaining in the chamber, 0.5 mL of the culture medium was newly added to the chamber. Furthermore, TFK-1 cells (purchased from RIKEN BioResource Center, RCB2537, Lot.002) suspended in 2.0 mL of RPMI 1640 culture medium containing 10% FBS, 100 units/mL penicillin, and 100 µg/mL streptomycin were seeded at 7 × 10⁴ cells/cm² in a dish having a diameter of 35 mm and cultured for 3 days, and therefore, monolayered cells were prepared in advance. After removing the culture medium in the dish, 0.5 mL of the culture medium for HepG2 cells was added thereto. The chamber was mounted on the monolayer of TFK-1 cells in the dish having a diameter of 35 mm, and then co-culture was carried out at 37°C for 1 hour in a CO₂ incubator (refer to Observation C of FIG. 5).
[4] The fluorescence intensities of Fluorescein in the culture medium in the chamber and in the dish were measured. Measurements were made three times respectively, and average values are shown in Table 1 below. In Table 1, FD (-) is a value measured as a background value using a chamber having HepG2 cells cultured without adding FD.

**[Table 1]**

| | Fluorescence intensity in chamber (average value) | FD (+) - FD (-) in chamber | Fluorescence intensity in dish (average value) | FD (+) - FD (-) in dish |
|---|---|---|---|---|
| FD (+) | 2478 | 1776 | 1224 | 511 |
| FD (-) | 702 | | 713 | |

Table 1 shows the fluorescence intensities of Fluorescein in the culture medium in the chamber and in the dish. Three pathways through which Fluorescein is excreted out of HepG2 cells were presumed, which are a pathway for discharge into the culture medium in the chamber, which is an upper side of HepG2 cells, a pathway for discharge into the culture medium in the dish where TFK-1 cells are present, which is a lower side of HepG2 cells, and a pathway for discharge by accumulation in a bile canaliculus-like structure between HepG2 cells and the bile canaliculus-like structure becoming open due to the presence of TFK-1 cells.

From Table 1, the fluorescence intensity of Fluorescein in the chamber is 1776, which shows the amount of Fluorescein discharged into the culture medium in the chamber which is the upper side of HepG2 cells. Furthermore, the fluorescence intensity of Fluorescein in the dish is 511, which shows the amount of Fluorescein discharged into the culture medium in the dish where TFK-1 cells are present which is the lower side of HepG2 cells.

Alternatively, from Observations B and C of FIG. 5, it was observed that Fluorescein accumulated in the bile canaliculus-like structure was excreted by co-culturing with TFK-1 cells, and therefore decreased. This indicates that co-culture with TFK-1 cells makes the bile canaliculus-like structure open (only the lower side, only the upper side, or both the lower side and the upper side).

Next, a change in the amount of Fluorescein excretion due to the presence or absence of TFK-1 cells was checked.

### [Example 2]

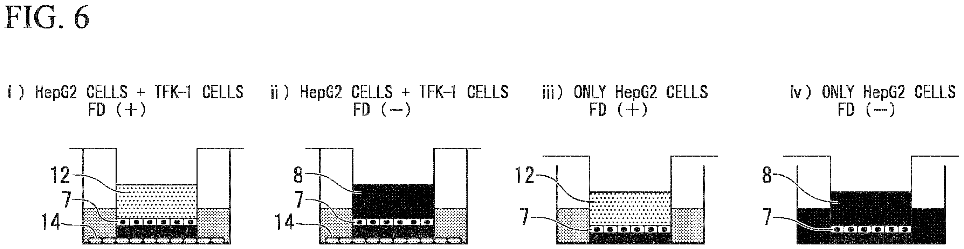

[1] HepG2 cells were seeded to prepare 12 sets of activated hydrogel membrane chambers. For 6 sets, FD was added to the culture medium and cultured by the same procedure as in [1] to [2] of Example 1.
[2] Subsequently, after removing the culture medium remaining in the chamber, 0.5 mL of the culture medium was newly added to the chamber. Subsequently, each of three sets was cultured at 37°C for 1 hour in a CO₂ incubator under the following four conditions (refer to FIG. 6).
   i) A set in which HepG2 cells were co-cultured with TFK-1 cells (FD added)
   ii) A set in which HepG2 cells were co-cultured with TFK-1 cells (FD not added)
   iii) A set in which HepG2 cells were homo-cultured in a dish containing only a culture medium (FD added)
   iv) A set in which HepG2 cells were homo-cultured in a dish containing only a culture medium (FD not added)
[3] The culture medium in the chamber and the dish of i) to iv) was recovered and the fluorescence intensities were measured. Measurements were made three times respectively, and average values were shown in Table 2 below.

**[Table 2]**

| Experimental condition | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 | i) FD (+) | 1 | 1287 | 1257 | 663 | 694 | 679 | 107 |
| cells + TFK-1 cells | | 2 | 1236 | | (86%) | 672 | | (14%) |
| | | 3 | 1249 | | | 671 | | |
| | ii) FD (-) | 4 | 576 | 594 | | 540 | 572 | |
| | | 5 | 610 | | | 578 | | |
| | | 6 | 597 | | | 597 | | |
| HepG2 cells only | iii) FD (+) | 7 | 1198 | 1145 | 549 (90%) | 665 | 645 | 60 (10%) |
| | | 8 | 1167 | | | 635 | | |
| | | 9 | 1071 | | | 636 | | |
| | iv) FD (-) | 10 | 591 | 596 | | 596 | 585 | |
| | | 11 | 595 | | | 583 | | |
| | | 12 | 603 | | | 576 | | |

Table 2 shows the fluorescence intensities of Fluorescein in the culture medium in the chamber and in the dish. For each of the set in which HepG2 cells were co-cultured with TFK-1 cells and the set in which HepG2 cells were homo-cultured in the dish containing the culture medium, the percentage of the value of FD (+) - FD (-) is shown in parentheses with a total of the amount of Fluorescein excreted into the chamber and the amount of Fluorescein excreted into the dish as 100%. For example, in the set in which HepG2 cells were co-cultured with TFK-1 cells, the amount of Fluorescein excreted into the chamber is calculated by 663 / (663 + 107) × 100, and is about 86 % of Fluorescein excreted from HepG2 cells.

From Table 2, in the set in which HepG2 cells were co-cultured with TFK-1 cells, a total of the fluorescence intensities of Fluorescein excreted into the chamber and into the dish is 770, whereas in the set in which HepG2 cells were homo-cultured in the dish containing the culture medium, a total of the fluorescence intensities of Fluorescein excreted into the chamber and into the dish is 609 and it became clear that the total amount of excreted Fluorescein is increased by about 26%.

It is presumed that this difference was attributable to the fact that in the set in which HepG2 cells were co-cultured with TFK-1 cells, excretion was enhanced by TFK-1 cells, and therefore excretion of Fluorescein remaining in the cytoplasm of HepG2 cells and Fluorescein accumulated in the bile canaliculus-like structure established between the cells of HepG2 cells (only the lower side, only the upper side, or both the lower side and the upper side) was enhanced.

Alternatively, it became clear from Table 2 that in the set in which HepG2 cells were co-cultured with TFK-1 cells, the amount of Fluorescein excreted into the dishes was 4% larger compared to that of the set in which HepG2 cells were homo-cultured in the dish containing the culture medium. Therefore, if it is assumed that a rate of Fluorescein excretion from the cytoplasm of HepG2 cells to the upper side (into the chamber) and the lower side (into the dish) does not change by co-culture with TFK-1 cells, it is reasonable to consider that the 4% improvement in the rate of Fluorescein excretion to the lower side by co-culture with TFK-1 cells is attributable to the fact that the excretion of Fluorescein accumulated in the bile canaliculus-like structure established between the cells of HepG2 cells is enhanced only to the lower side.

### [Example 3]

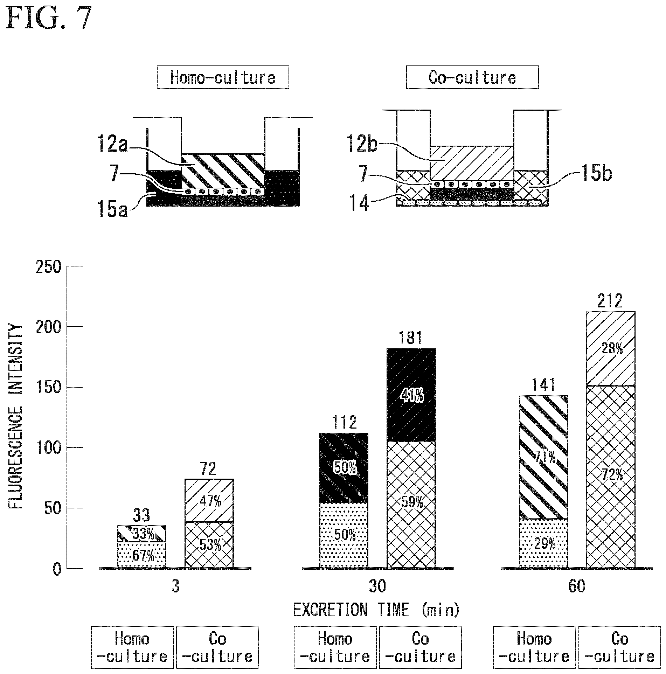

[1] HepG2 cells were seeded to prepare 36 sets of activated hydrogel membrane chambers. For 18 sets, FD was added to the culture medium and cultured by the same procedure as in [1] to [2] of Example 1.
[2] With respect to the 36 sets of hydrogel membrane chambers in which the cells were seeded and cultured in [1], the culture medium remaining in the chamber was removed, and then 0.5 mL of the culture medium was newly added to the chamber. Subsequently, each of three sets was cultured at 37°C in a CO₂ incubator with culture time of 3 minutes, 30 minutes, and 60 minutes (for each 12 sets) under the following four conditions i) to iv) the same as those of Example 2.
[3] The culture media in the dishes and in the chambers of i) to iv) were recovered 3 minutes, 30 minutes, and 60 minutes after the start of culture, and the fluorescence intensities were measured. Measurements were made three times respectively, and average values are shown in Table 3 (culture time 3 minutes), Table 4 (culture time 30 minutes), and Table 5 (culture time 60 minutes). Furthermore, Table 3 to Table 6 are summarized in a graph and shown in FIG. 7. In FIG. 7, the term "Co-culture" means the set in which HepG2 cells were co-cultured with TFK-1 cells, and the term "Homo-culture" means the set in which HepG2 cells were homo-cultured in the dish containing the culture medium.

**[Table 3]**

| Experimental condition (culture time 3 minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 1 | 520 | 525 | 34 (47%) | 512 | 526 | 38 (53%) |
| | | 2 | 538 | | | 548 | | |
| | | 3 | 518 | | | 517 | | |
| | ii) FD (-) | 4 | 479 | 491 | | 471 | 488 | |
| | | 5 | 494 | | | 501 | | |
| | | 6 | 499 | | | 491 | | |
| HepG2 cells only | iii) FD (+) | 7 | 511 | 507 | 11 (33%) | 506 | 530 | 22 (67%) |
| | | 8 | 508 | | | 550 | | |
| | | 9 | 502 | | | 533 | | |
| | iv) FD (-) | 10 | 499 | 496 | | 499 | 508 | |
| | | 11 | 488 | | | 517 | | |
| | | 12 | 500 | | | 507 | | |

**[Table 4]**

| Experimental condition (culture time 30 ) minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 13 | 555 | 580 | 75 (41%) | 559 | 581 | 106 (59%) |
| | | 14 | 567 | | | 620 | | |
| | | 15 | 617 | | | 563 | | |
| | ii) FD (-) | 16 | 506 | 505 | | 469 | 475 | |
| | | 17 | 499 | | | 478 | | |
| | | 18 | 509 | | | 478 | | |
| HepG2 cells only | iii) FD (+) | 19 | 523 | 519 | 56 (50%) | 507 | 515 | 56 (50%) |
| | | 20 | 516 | | | 522 | | |
| | | 21 | 517 | | | 515 | | |
| | iv) FD (-) | 22 | 461 | 463 | | 456 | 459 | |
| | | 23 | 468 | | | 466 | | |
| | | 24 | 459 | | | 456 | | |

**[Table 5]**

| Experimental condition (culture time 60 ) minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 25 | 487 | 507 | 59 (28%) | 639 | 599 | 153 (72%) |
| | | 26 | 499 | | | 572 | | |
| | | 27 | 535 | | | 585 | | |
| | ii) FD (-) | 28 | 438 | 448 | | 428 | 446 | |
| | | 29 | 455 | | | 461 | | |
| | | 30 | 452 | | | 450 | | |
| HepG2 cells only | iii) FD (+) | 31 | 574 | 582 | 100 (71%) | 513 | 520 | 41 (29%) |
| | | 32 | 565 | | | 516 | | |
| | | 33 | 606 | | | 531 | | |
| | iv) FD (-) | 34 | 484 | 482 | | 468 | 479 | |
| | | 35 | 468 | | | 484 | | |
| | | 36 | 494 | | | 486 | | |

Tables 3 to 5 show the fluorescence intensities of Fluorescein in the culture medium in the chamber and in the dish, similarly to Table 2. For each of the set in which HepG2 cells were co-cultured with TFK-1 cells and the set in which HepG2 cells were homo-cultured in the dish containing the culture medium, the percentage of the value of FD (+) - FD (-) is shown in parentheses with a total of the amount of Fluorescein excreted into the chamber and the amount of Fluorescein excreted into the dish as 100%.

From Tables 3 to 5 and FIG. 7, it became clear that in the set in which HepG2 cells were co-cultured with TFK-1 cells, the total of the fluorescence intensities of excreted Fluorescein was already larger at the point of the culture time of 3 minutes than the total of the fluorescence intensities of Fluorescein excreted into the chamber and into the dish of the set in which HepG2 cells were homo-cultured in the dish containing the culture medium. In addition, it became clear that also at the culture time of 30 minutes and 60 minutes, in the set in which HepG2 cells were co-cultured with TFK-1 cells, the total of the fluorescence intensities of Fluorescein excreted into the chamber and into the dish was larger than the total of the fluorescence intensities of Fluorescein excreted into the chamber and into the dish of the set in which HepG2 cells were homo-cultured in the dish containing the culture medium.

From FIG. 7, in both the set in which HepG2 cells were co-cultured with TFK-1 cells and the set in which HepG2 cells were homo-cultured in the dish containing the culture medium, the total of the fluorescence intensities of Fluorescein excreted into the chamber and into the dish was increased over time. It became clear that the total of the fluorescence intensities of Fluorescein excreted into the chamber and into the dish of the set in which HepG2 cells were homo-cultured in the dish containing the culture medium becomes almost a plateau state when the culture time exceeds 30 minutes.

Furthermore, in the set in which HepG2 cells were co-cultured with TFK-1 cells, the rate of the fluorescence intensities of Fluorescein excreted into the dish (lower side) was increased, whereas in the set in which HepG2 cells were homo-cultured in the dish containing the culture medium, the rate of the fluorescence intensities of Fluorescein excreted into the dish (lower side) was decreased over time. In addition, it became clear that at the culture time of 60 minutes, in the set in which HepG2 cells were co-cultured with TFK-1 cells, the amount of Fluorescein excreted into the dish (lower side) was 43% larger than that of the set in which HepG2 cells were homo-cultured in the dish containing the culture medium.

Therefore, similarly to Example 2, if it is assumed that the rate of Fluorescein excretion from the cytoplasm of HepG2 cells to the upper side (into the chamber) and the lower side (into the dish) does not change by co-culture with TFK-1 cells, it is reasonable to consider that the 46% improvement in the rate of Fluorescein excretion to the lower side by co-culture with TFK-1 cells is attributable to the fact that the excretion of Fluorescein accumulated in the bile canaliculus-like structure established between the cells of HepG2 cells is enhanced only to the lower side.

### [Example 4]

An experiment separate from Example 3 was conducted in order to examine whether or not the excretion amount of Fluorescein continues to increase in the set in which HepG2 cells were co-cultured with TFK-1 cells by prolonging the culture time.

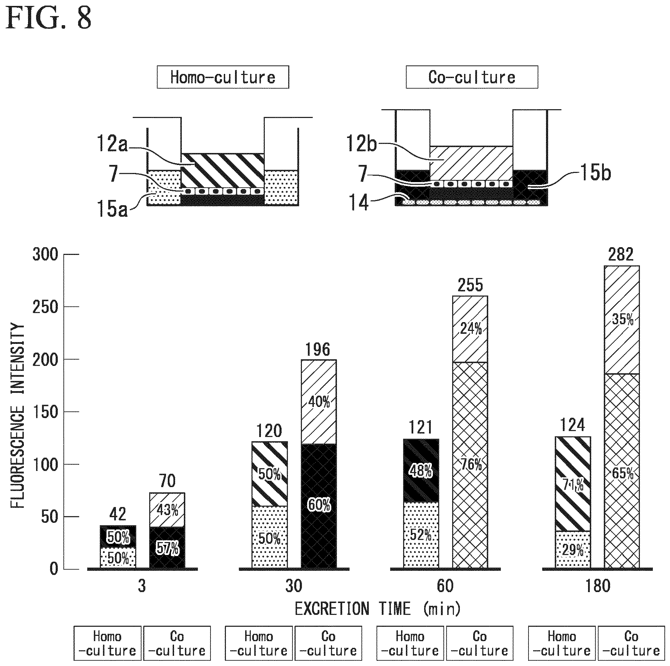

[1] First, HepG2 cells were seeded to prepare 48 sets of activated hydrogel membrane chambers. For 24 sets, FD was added to the culture medium and cultured by the same procedure as in [1] to [2] of Example 1.
[2] With respect to the 48 sets of hydrogel membrane chambers in which the cells were seeded and cultured in [1], the culture medium remaining in the chamber was removed, and then 0.5 mL of the culture medium was newly added to the chamber. Subsequently, each of three sets was cultured at 37°C in a CO₂ incubator with culture time of 3 minutes, 30 minutes, 60 minutes, and 180 minutes (for each 12 sets) under the following four conditions i) to iv) the same as those of Example 2.
[3] The culture media in the dishes and in the chambers of i) to iv) were recovered 3 minutes, 30 minutes, 60 minutes, and 180 minutes after the start of culture, and the fluorescence intensities were measured. Measurements were made three times respectively, and average values are shown in Table 6 (culture time 3 minutes), Table 7 (culture time 30 minutes), Table 8 (culture time 60 minutes), and Table 9 (culture time 180 minutes). Furthermore, Table 6 to Table 9 are summarized in a graph and shown in FIG. 8. In FIG. 8, the term "Co-culture" means the set in which HepG2 cells were co-cultured with TFK-1 cells, and the term "Homo-culture" means the set in which HepG2 cells were homo-cultured in the dish containing the culture medium.

**[Table 6]**

| Experimental condition (culture time 3 minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 1 | 524 | 516 | 30 (43%) | 532 | 529 | 40 (57%) |
| | | 2 | 519 | | | 534 | | |
| | | 3 | 504 | | | 520 | | |
| | ii) FD (-) | 4 | 488 | 486 | | 470 | 489 | |
| | | 5 | 481 | | | 505 | | |
| | | 6 | 489 | | | 491 | | |
| HepG2 cells only | iii) FD (+) | 7 | 509 | 507 | 21 (50%) | 506 | 508 | 21 (50%) |
| | | 8 | 505 | | | 511 | | |
| | | 9 | 506 | | | 507 | | |
| | iv) FD (-) | 10 | 485 | 486 | | 469 | 487 | |
| | | 11 | 487 | | | 493 | | |
| | | 12 | 485 | | | 499 | | |

**[Table 7]**

| Experimental condition (culture time 30 minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 13 | 567 | 582 | 78 (40%) | 591 | 615 | 118 (60%) |
| | | 14 | 589 | | | 604 | | |
| | | 15 | 590 | | | 650 | | |
| | ii) FD (-) | 16 | 506 | 504 | | 499 | 497 | |
| | | 17 | 505 | | | 491 | | |
| | | 18 | 502 | | | 500 | | |
| HepG2 cells only | iii) FD (+) | 19 | 538 | 537 | 60 (50%) | 530 | 530 | 60 (50%) |
| | | 20 | 527 | | | 548 | | |
| | | 21 | 545 | | | 511 | | |
| | iv) FD (-) | 22 | 470 | 477 | | 450 | 470 | |
| | | 23 | 473 | | | 494 | | |
| | | 24 | 487 | | | 467 | | |

**[Table 8]**

| Experimental condition (culture time 60 minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 25 | 560 | 550 | 61 (24%) | 674 | 666 | 194 (76%) |
| | | 26 | 549 | | | 667 | | |
| | | 27 | 541 | | | 657 | | |
| | ii) FD (-) | 28 | 490 | 489 | | 465 | 472 | |
| | | 29 | 486 | | | 480 | | |
| | | 30 | 490 | | | 471 | | |
| HepG2 cells only | iii) FD (+) | 31 | 559 | 556 | 58 (48%) | 518 | 548 | 63 (52%) |
| | | 32 | 568 | | | 582 | | |
| | | 33 | 542 | | | 545 | | |
| | iv) FD (-) | 34 | 496 | 498 | | 478 | 485 | |
| | | 35 | 503 | | | 482 | | |
| | | 36 | 495 | | | 494 | | |

**[Table 9]**

| Experimental condition (culture time 180 minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 37 | 572 | 589 | 99 (35%) | 671 | 664 | 183 (65%) |
| | | 38 | 595 | | | 659 | | |
| | | 39 | 600 | | | 661 | | |
| | ii) FD (-) | 40 | 486 | 490 | | 480 | 481 | |
| | | 41 | 490 | | | 480 | | |
| | | 42 | 495 | | | 483 | | |
| HepG2 cells only | iii) FD (+) | 43 | 567 | 579 | 88 (71%) | 515 | 515 | 36 (29%) |
| | | 44 | 601 | | | 509 | | |
| | | 45 | 568 | | | 522 | | |
| | iv) FD (-) | 46 | 499 | 491 | | 461 | 479 | |
| | | 47 | 484 | | | 485 | | |
| | | 48 | 490 | | | 491 | | |

Tables 6 to 9 show the fluorescence intensities of Fluorescein in the culture medium in the chamber and in the dish, similarly to Table 2. For each of the set in which HepG2 cells were co-cultured with TFK-1 cells and the set in which HepG2 cells were homo-cultured in the dish containing the culture medium, the percentage of the value of FD (+) - FD (-) is shown in parentheses with a total of the amount of Fluorescein excreted into the chamber and the amount of Fluorescein excreted into the dish as 100%.

From Tables 6 to 9 and FIG. 8, it became clear that at any time of the culture time, in the set in which HepG2 cells were co-cultured with TFK-1 cells, the total of the fluorescence intensities of Fluorescein excreted into the chamber and into the dish was larger than the total of the fluorescence intensities of Fluorescein excreted into the chamber and into the dish of the set in which HepG2 cells were homo-cultured in the dish containing the culture medium.

From FIG. 8, in both the set in which HepG2 cells were co-cultured with TFK-1 cells and the set in which HepG2 cells were homo-cultured in the dish containing the culture medium, the total of the fluorescence intensities of Fluorescein excreted into the chamber and into the dish was increased over time. Similarly to Example 3, the total of the fluorescence intensities of Fluorescein excreted into the chamber and into the dish of the set in which HepG2 cells were homo-cultured in the dish containing the culture medium becomes almost a plateau state when the culture time exceeds 30 minutes.

In addition, it became clear that the total of the fluorescence intensities of Fluorescein excreted into the chamber and into the dish of the set in which HepG2 cells were co-cultured with TFK-1 cells becomes almost a plateau state when the culture time exceeds 60 minutes.

Furthermore, in the set in which HepG2 cells were co-cultured with TFK-1 cells, the rate of the fluorescence intensities of Fluorescein excreted into the dish (lower side) was increased, whereas in the set in which HepG2 cells were homo-cultured in the dish containing the culture medium, the rate of the fluorescence intensities of Fluorescein excreted into the dish (lower side) was decreased over time. In addition, it became clear that at the culture time of 180 minutes, in the set in which HepG2 cells were co-cultured with TFK-1 cells, the amount of Fluorescein excreted into the dish (lower side) was 36% larger compared to that of the set in which HepG2 cells were homo-cultured in the dish containing the culture medium.

Therefore, similarly to Example 2 and Example 3, if it is assumed that the rate of Fluorescein excretion from the cytoplasm of HepG2 cells to the upper side (into the chamber) and the lower side (into the dish) does not change by co-culture with TFK-1 cells, it is reasonable to consider that the 36% improvement in the rate of Fluorescein excretion to the lower side by co-culture with TFK-1 cells is attributable to the fact that the excretion of Fluorescein accumulated in the bile canaliculus-like structure established between the cells of HepG2 cells is enhanced only to the lower side.

From the results of Examples 2 to 4, it became clear that the excretion of the candidate compound sensitive to excretion into bile or blood with the passage of time can be observed over time using the evaluation method of the present invention.

Therefore, it can be presumed that the evaluation method of the present invention can be expanded to application research of evaluation of the candidate compound sensitive to excretion into bile which repeats the enterohepatic circulation.

### [Example 5]

An experiment was conducted with respect to the set in which HepG2 cells were co-cultured with TFK-1 cells in order to examine an excretion timing of Fluorescein.

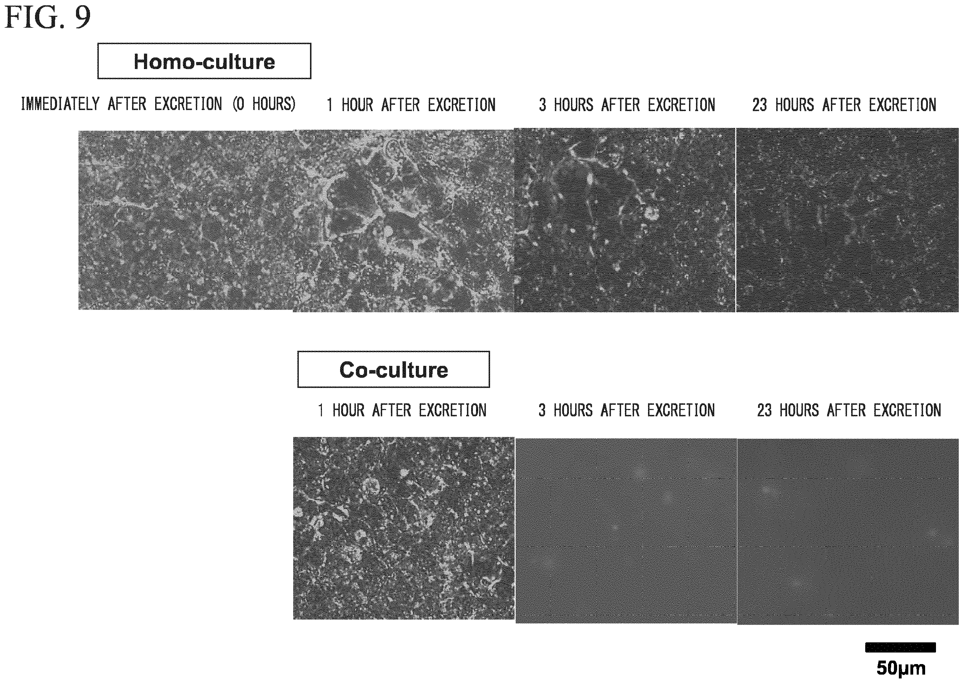

[1] First, HepG2 cells were seeded to prepare 2 sets of activated hydrogel membrane chambers. FD was added to the culture medium and cultured by the same procedure as in [1] to [2] of Example 1.
[2] With respect to the 2 sets of hydrogel membrane chambers in which the cells were seeded and cultured in [1], the culture medium remaining in the chamber was removed, and then 0.5 mL of the culture medium was newly added to the chamber. Subsequently, with respect to each set, changes in Fluorescein remaining in the cytoplasm of HepG2 cells and Fluorescein accumulated in the bile canaliculus-like structure established between the cells of HepG2 cells at 0 hours (immediately after excretion), 1 hour, 3 hours, and 23 hours after the start of culture (excretion start) were observed with a fluorescence microscope over time under the conditions i) and iii) described in Example 2. The results are shown in FIG. 9. In FIG. 9, the term "Co-culture" means the set in which HepG2 cells were co-cultured with TFK-1 cells, and the term "Homo-culture" means the set in which HepG2 cells were homo-cultured in the dish containing the culture medium.

From FIG. 9, it is possible to confirm abundant green fluorescence of Fluorescein in the bile canaliculus-like structure and the cytoplasm of a few cells immediately after the start of culture, that is, immediately after that excretion (0 hours). After 1 hour of excretion, reduction in the green fluorescence of Fluorescein was not noticeable in the homo-culture, whereas it was significantly noticeable in the co-culture. But in both the homo-culture and the co-culture, it was possible to confirm the green fluorescence of Fluorescein not only in the bile canaliculus-like structure but also in the cytoplasm of very few cells. It was not possible to confirm the green fluorescence of Fluorescein in the co-culture after 3 hours of excretion and after 23 hours of excretion. In the homo-culture, the reduction in the green fluorescence of Fluorescein was significantly noticeable after 3 hours of excretion, and it was possible to confirm the green fluorescence of Fluorescein in the bile canaliculus-like structure and the cytoplasm of very few cells. In the homo-culture after 23 hours of excretion, the reduction in the green fluorescence of Fluorescein was accelerated and it was not possible to confirm the fluorescence in the cytoplasm, but it was confirmed that the fluorescence in the bile canaliculus-like structure remained.

Therefore, it is considered that in the case of the homo-culture, it needs 1 day or more to reduce the amount of Fluorescein accumulated in the bile canaliculus-like structure and the bile canaliculus-like structure retains almost a closed structure. In addition, it is considered that the bile canaliculus-like structure was opened in the case of the co-culture from the fact that the reduction in the amount of Fluorescein accumulated in the bile canaliculus-like structure was confirmed within 1 hour. From the results of the co-culture of FIG. 7 and FIG. 8, it is considered that most part of the opening of this bile canaliculus-like structure is on the lower side where TFK-1 cells are present.

### [Example 6]

An experiment was conducted in order to examine the excretion amount of Fluorescein in a case of using cells other than TFK-1 cells to be cultured in the dish.

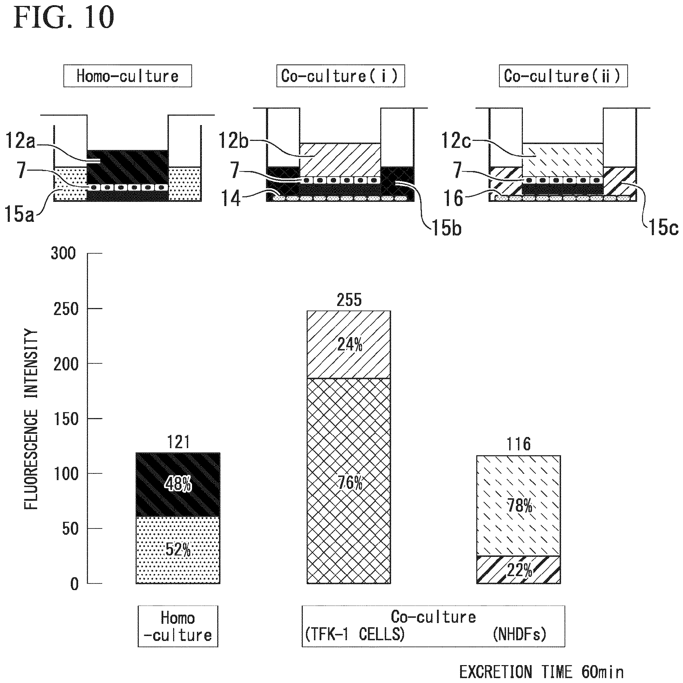

[1] First, HepG2 cells were seeded to prepare 18 sets of activated hydrogel membrane chambers. For 9 sets, FD was added to the culture medium and cultured by the same procedure as in [1] to [2] of Example 1.
[2] With respect to the 18 sets of hydrogel membrane chambers in which the cells were seeded and cultured in [1], the culture medium remaining in the chamber was removed, and then 0.5 mL of the culture medium was newly added to the chamber. Subsequently, each of three sets was cultured at 37°C for 1 hour in a CO₂ incubator under the following four conditions i) to iv) the same as those of Example 2, and the additional conditions v) and vi) described below.
   v) A set in which HepG2 cells were co-cultured with Normal Human Dermal Fibroblasts (NHDFs) (FD added)
   vi) A set in which HepG2 cells were co-cultured with NHDFs (FD not added)
[3] The culture media in the dishes and in the chambers of i) to vi) were recovered and the fluorescence intensities were measured. Measurements were made three times respectively, and average values are shown in Table 10. Furthermore, Table 10 is summarized in a graph and shown in FIG. 10. In FIG. 10, the term "Co-culture (i)" means the set in which HepG2 cells were co-cultured with TFK-1 cells, the term "Co-culture (ii)" means the set in which HepG2 cells were co-cultured with NHDFs, and the term "Homo-culture" means the set in which HepG2 cells were cultured in the dish containing the homo-culture medium.

**[Table 10]**

| Experimental condition (culture time 60 minutes) | | No. | In chamber | | | In dish | | |
|---|---|---|---|---|---|---|---|---|
| | | | Fluorescence intensity (average value) | Average value of set | FD (+) - FD (-) | Fluorescence intensity (average value) | Average value of set | FD (+) - FD (-) |
| HepG2 cells + TFK-1 cells | i) FD (+) | 1 | 560 | 550 | 61 (24%) | 674 | 666 | 194 (76%) |
| | | 2 | 549 | | | 667 | | |
| | | 3 | 541 | | | 657 | | |
| | ii) FD (-) | 4 | 490 | 489 | | 465 | 472 | |
| | | 5 | 486 | | | 480 | | |
| | | 6 | 490 | | | 471 | | |
| HepG2 cells only | iii) FD (+) | 7 | 559 | 556 | 58 (48%) | 518 | 548 | 63 (52%) |
| | | 8 | 568 | | | 582 | | |
| | | 9 | 542 | | | 545 | | |
| | iv) FD (-) | 10 | 496 | 498 | | 478 | 485 | |
| | | 11 | 503 | | | 482 | | |
| | | 12 | 495 | | | 494 | | |
| HepG2 cells + NHDFs | v) FD (+) | 13 | 569 | 569 | 90 (78%) | 502 | 500 | 26 (22%) |
| | | 14 | 578 | | | 496 | | |
| | | 15 | 560 | | | 502 | | |
| | vi) FD (-) | 16 | 479 | 479 | | 470 | 474 | |
| | | 17 | 484 | | | 482 | | |
| | | 18 | 475 | | | 470 | | |

From Table 10 and FIG. 10, the total of the fluorescence intensities of Fluorescein excreted into the chamber and into the dish in the set in which HepG2 cells were co-cultured with NHDFs was almost the same as the set in which HepG2 cells were homo-cultured in the dish containing the culture medium. In addition, it became clear that the amount of Fluorescein excreted into the dish in the set in which HepG2 cells were co-cultured with NHDFs was 54% smaller than the amount of Fluorescein excreted into the dish in the set in which HepG2 cells were co-cultured with TFK-1 cells. Therefore, this indicates that it is preferable to select cells such as TFK-1 cells, which has an effect of enhancing excretion from hepatocytes, as cells for co-culture.

### [Example 7]

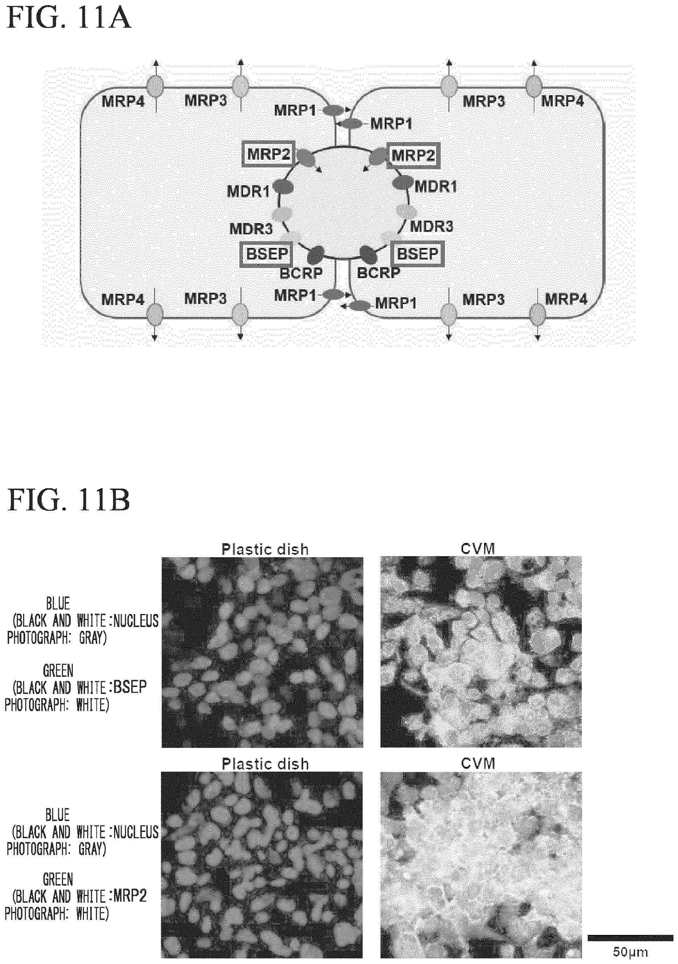

In order to confirm that the bile canaliculus-like structure is formed in the hepatocyte culture device of the present invention, the presence or absence of a transporter (MRP2 (multidrug resistance-associated protein 2) and BSEP (bile salt export pump)) which is localized (internalized into the cytoplasm in some cases) in the bile canalicular membrane was evaluated (refer to FIG. 11A).

[1] First, a set in which HepG2 cells were seeded directly in the dish and a set in which HepG2 cells were seeded in a hydrogel membrane chamber to be activated were prepared.
[2] Subsequently, MRP2 and BSEP, which are the transporters localized (internalized into the cytoplasm in some cases) in the bile canalicular membrane, were subjected to immunostaining. As a primary antibody, a mouse anti-MRP2 antibody (manufactured by Santa Cruz Biotechnology, Inc.) or a goat anti-BSEP antibody (manufactured by Santa Cruz Biotechnology, Inc.) was used, and as a secondary antibody, Fluorescein-conjugated goat anti-mouse IgG antibody (manufactured by Santa Cruz Biotechnology, Inc.) or Fluorescein-conjugated donkey anti-goat IgG antibody (manufactured by Santa Cruz Biotechnology, Inc.), which exhibits green fluorescence was used. As a counterstain, nuclei were stained using Hoechst 33342 (manufactured by DOJINDO LABORATORIES), which exhibits blue fluorescence. The results are shown in FIG. 11B. In FIG. 11B, "Plastic dish" means the set in which the HepG2 cells were seeded directly in the dish, and "CVM" means the set in which the HepG2 cells were seeded in the hydrogel membrane chamber to be activated. In FIG. 11B, in the black and white photograph, the blue-strained nucleus is gray and the green-strained BSEP and MRP2 are white.

From FIG. 11B, the fluorescence of BSEP and MRP2 was not detected in the set in which the HepG2 cells were seeded directly in the dish, and fluorescence of BSEP and MRP2 was detected in the set in which the HepG2 cells were seeded in the hydrogel membrane chamber to be activated. Therefore, it was possible to confirm that MRP2 and BSEP were expressed as a transporter involved in biliary excretion in the set in which the HepG2 cells were seeded in the hydrogel membrane chamber to be activated.

From the above, it is clear that it is possible to provide a hepatocyte culture device that enhances accumulation and excretion of a hepatic metabolite in and into the bile canaliculus-like structure according to the present invention.

### Industrial Applicability

According to the hepatocyte culture device of the present invention, it is possible to obtain a substance metabolized by hepatocytes in the environment reflecting in vivo in a short period of time at low cost. It is possible to collectively recover hepatic metabolites excreted into the bile canaliculus-like structure established between the hepatocytes without destroying tight junctions between the hepatocytes.

According to the method for evaluating the candidate compound sensitive to excretion into bile or blood, and the hepatic metabolite of the candidate compound using the hepatocyte culture device of the present invention, it is possible to evaluate types and amount of hepatic metabolite metabolized and excreted into bile or blood by activated drug-metabolizing enzymes, and to further evaluate hepatotoxicity of the candidate compound and the hepatic metabolite of the candidate compound in a short period of time at low cost.

### Reference Signs List

1 tubular carrier
2 membrane through which physiologically active substance can pass
3 film
4 locking portion
5 hydrogel membrane
6 silicone-coated PET film
7 HepG2 cells
8 DMEM culture medium containing 10% FBS, 20 mM HEPES, 100 units/mL penicillin, and 100 µg/mL streptomycin
9 well of 12-well plate
10 bile canaliculus-like structure
11 tight junction
12 culture medium described in 8 which contains FD
12a culture medium described in 8 which contains FD excreted into upper side in homo-culture of HepG2 cells alone
12b culture medium described in 8 which contains FD excreted into upper side in co-culture of HepG2 cells with TFK-1 cells
12c culture medium described in 8 which contains FD excreted into upper side in co-culture of HepG2 cells with HDF cells
13 petri dish
14 TFK-1 cells
15a culture medium described in 8 which contains FD excreted into lower side in homo-culture of HepG2 cells alone
15b culture medium described in 8 which contains FD excreted into lower side in co-culture of HepG2 cells with TFK-1 cells
15c culture medium described in 8 which contains FD excreted into lower side in co-culture of HepG2 cells with HDF cells
16 NHDFs (normal human dermal fibroblasts)
X portable culture vessel
Y hydrogel membrane chamber

## Claims

1. A hepatocyte culture device enhancing accumulation and excretion of a hepatic metabolite in and into a bile canaliculus-like structure, the device comprising:
a first cell culture containing a plurality of hepatocytes;
a portable culture vessel in which the bile canaliculus-like structure can be established between the hepatocytes in the first cell culture, and which has, on an outer surface of the culture vessel, a membrane through which a physiologically active substance can pass; and
a second cell culture capable of improving excretion activity of a hepatic metabolite in the first cell culture,
wherein the first cell culture cultured in the portable culture vessel is disposed on the second cell culture to be co-cultured.

2. The hepatocyte culture device according to Claim 1,
wherein the portable culture vessel improves metabolic activity of the first cell culture.

3. The hepatocyte culture device according to Claim 1 or 2,
wherein the excretion activity of the hepatic metabolite is activity of excreting the hepatic metabolite accumulated in the bile canaliculus-like structure between the hepatocytes and in the hepatocytes in the first cell culture.

4. The hepatocyte culture device according to any one of Claims 1 to 3,
wherein the hepatocytes are cultured hepatocytes derived from normal liver tissues, liver cancer tissues, or stem cells which are selected from the group consisting of humans, rats, monkeys, apes, cats, dogs, pigs, cows, sheep, horses, chickens, and ducks.

5. The hepatocyte culture device according to any one of Claims 1 to 4,
wherein the second cell culture is a cultured cell derived from an epithelium, mesenchyme, or an endothelium which are selected from the group consisting of humans, rats, monkeys, apes, cats, dogs, pigs, cows, sheep, horses, chickens, and ducks, and is a cell selected from the group consisting of intrahepatic bile duct epithelial cells, extrahepatic bile duct epithelial cells, vascular endothelial cells, fibroblasts, and culture supernatant fluids of these cells.

6. The hepatocyte culture device according to any one of Claims 1 to 5,
wherein the membrane through which a physiologically active substance can pass contains an extracellular matrix component to induce a gelation.

7. The hepatocyte culture device according to Claim 6,
wherein the extracellular matrix component to induce a gelation is collagen.

8. A method for evaluating a candidate compound sensitive to excretion into bile or blood, and a hepatic metabolite of the candidate compound using hepatocytes,
wherein a first cell culture containing a plurality of hepatocytes,
a portable culture vessel in which a bile canaliculus-like structure can be established between the hepatocytes in the first cell culture, and that has, on an outer surface of the culture vessel, a membrane through which a physiologically active substance can pass, and
a second cell culture capable of improving excretion activity of a hepatic metabolite in the first cell culture, are included,
the method comprising:
(a) a step of exposing the candidate compound to the first cell culture cultured in the portable culture vessel;
(b) a step of culturing the first cell culture to which the candidate compound is exposed by disposing the culture on each of a vessel containing only a culture medium and a vessel containing the second cell culture;
(c) a step of checking a change in the bile canaliculus-like structure of the first cell culture, and recovering a substance excreted into the portable culture vessel and a substance excreted into each of the vessel containing only the culture medium and the vessel containing the second cell culture; and
(d) a step of analyzing the recovered substances for an excreted hepatic metabolite and an amount thereof.

9. The evaluation method according to Claim 8,
wherein the portable culture vessel improves metabolic activity of the first cell culture.

10. The evaluation method according to Claim 8 or 9,
wherein the excretion activity of the hepatic metabolite is activity of excreting the hepatic metabolite accumulated in the bile canaliculus-like structure between the hepatocytes and in the hepatocytes in the first cell culture.
